# EUROPÄISCHE PATENTSCHRIFT

(11) **EP 3 252 033 B1**
(45) Veröffentlichungstag und Bekanntmachung des Hinweises auf die Patenterteilung: **27.06.2018**
(21) Anmeldenummer: 17173890.9
(22) Anmeldetag: 01.06.2017
(51) Int. Cl.: C07C 209/18, C25B 3/10, C07C 211/58, C07C 215/86, C07C 217/94, C07C 213/08, C07C 217/78, C25B 11/12

(54) **ZWEISTUFIGE SYNTHESE VON N-BIARYLVERBINDUNGEN**
TWO-STAGE SYNTHESIS OF N BIARYL COMPOUNDS
SYNTHÈSE EN DEUX ÉTAPES DE COMPOSÉS DE N-BIARYL

(30) Priorität: 03.06.2016 DE 102016209814
(43) Veröffentlichungstag der Anmeldung: 06.12.2017
(73) Patentinhaber: Evonik Degussa GmbH, 45128 Essen (DE)
(72) Erfinder: Dyballa, Katrin Marie, 45657 Recklinghausen (DE); Franke, Robert, 45772 Marl (DE); Fridag, Dirk, 45721 Haltern am See (DE); Dahms, Benedikt, 55116 Mainz (DE); Waldvogel, Siegfried R., 55435 Gau-Algesheim (DE)

(56) Entgegenhaltungen:
- WO-A1-2014/135371
- MATSUMOTO, K.; YOSHIDA, M.; SHINDO, M.: "Heterogeneous Rhodium-Catalyzed Aerobic Oxidative Dehydrogenative Cross-Coupling: Nonsymmetrical Biaryl Amines", ANGEWANDTE CHEMIE, Bd. 55, 21. März 2016 (2016-03-21), Seiten 5272-5276, XP002774333,
- PIRKLE W H ET AL: "PREPARATION OF N-(2-NAPHTHYL)-2-AMINO ACIDS AND ESTERS OF HIGH ENANTIOMERIC PURITY", THE JOURNAL OF ORGANIC CHEMISTRY, AMERICAN CHEMICAL SOCIETY, US, Bd. 51, Nr. 1, 1. Januar 1986 (1986-01-01) , Seiten 102-105, XP002061135, ISSN: 0022-3263, DOI: 10.1021/JO00351A024

## Beschreibung

Die folgende Erfindung betrifft eine zweistufige Synthese von N-Biarylverbindungen, sowie N-Biarylverbindungen, welche mit Hilfe der neuen Syntheseroute hergestellt wurden.

Die Darstellung chiraler Naphthylaminderivate mittels Bucherer-Reaktion sowie das elektrochemische Verfahren zur Kreuzkupplung von ungeschützten Phenolen mit chiralen Naphthylaminderivaten sind bisher nicht literaturbekannt. Lediglich die direkte Kreuzkupplung ungeschützter Phenole mit nicht chiralen Naphthylaminderivate ist bisher für nur sehr wenige Beispiele beschrieben. Hierzu ist jedoch die Verwendung von Rhodium als Katalysator sowie TFA als Lösungsmittel notwendig (K. Matsumoto, M. Yoshida, M. Shindo, Angew. Chem. Int. Ed, 2016, 55, 5272-5276).

Andere Syntheserouten zur Darstellung unsymmetrischer Biarylverbindungen umfassten entweder das Schützen der Aminogruppe verschiedener Arylamine vor der oxidativen Kreuzkupplung oder die nachträgliche Funktionalisierung jener, sowie die überstöchiometrische Verwendung von Oxidationsmitteln wie z.B. Fe(III) (K. Ding, H. Guo, X. Li, Y. Yuan, Y. Wang, Top Catal, 2005, 35, 105-116; G.-Q. Li, H. Gao, C. Keene, M. Devonas, D. H. Ess, L. Kürti, J. Am. Chem. Soc. 2013, 135, 7414-7417).

Ein großer Nachteil der oben genannten Methoden zur Darstellung chiraler Naphthylaminederivate sowie der Phenol-Naphthylamin-Kreuzkupplung ist die häufige Notwendigkeit trockener Lösungsmittel und eines Luftausschlusses. Weiterhin werden oft große Mengen teils giftiger Oxidationsmittel verwendet. Während der Reaktion treten oft toxische Nebenprodukte auf, die vom gewünschten Produkt aufwendig abgetrennt und teuer entsorgt werden müssen. Durch knapper werdende Rohstoffe (z.B. Bor und Brom im Falle der übergangsmetallkatalysierten Kreuzkupplung) und die steigende Relevanz des Umweltschutzes steigt der Preis für solche Transformationen. Vor allem bei der Nutzung von mehrstufigen Sequenzen ist ein Wechsel von verschiedenen Lösungsmitteln notwendig.

Die Aufgabe der folgenden Erfindung bestand darin, ein Verfahren bereitzustellen, bei dem chirale Naphthylaminderivate hergestellt werden können, und welches die im Stand der Technik beschriebenen Nachteile nicht aufweist.

Die Aufgabe wird gelöst durch ein Verfahren nach Anspruch 1.

Verfahren umfassend die Verfahrensschritte:
a) Umsetzung eines Naphthols, welches in ortho-Position zur OH-Gruppe einen Wasserstoff aufweist, mit einem chiralen primären Amin in Gegenwart von Wasser und unter Zusatz von Natriumhydrogensulfit, so dass ein sekundäres Naphthylaminderivate entsteht,
b) elektrochemische Kupplung des sekundären Naphthylaminderivats aus a) unter Verwendung eines Lösungsmittels und eines Leitsalzes mit einem Phenol, welches in ortho-Position zur OH-Gruppe einen Wasserstoff aufweist, so dass
   - entweder eine direkte Kupplung des Phenylrings des Phenols in ortho-Position zur OH-Gruppe mit dem Naphthylringsystem in ortho-Positon zur Aminogruppe erfolgt, oder
   - der Sauerstoff des Phenols an das Naphthylringsystem in ortho-Positon zur Aminogruppe gekuppelt wird.

Die oder-Verknüpfung der beiden Kupplungsvarianten ist so zu verstehen, dass bei der Reaktion eines Naphthylaminderivats-Moleküs mit einem Phenol-Molekül nur eine der beiden Kupplungen stattfindet. In einem Reaktionsansatz / -gefäß, welcher eine Vielzahl von Molekülen aufweist, können beide Kupplungsvarianten nebeneinander ablaufen.

Durch die Erfindung wird eine einfache und kosteneffiziente Methode zur Darstellung chiraler Naphthylaminderivate unter Verwendung der Bucherer-Reaktion etabliert. Die erhaltenen Derivate können im Anschluss mit unterschiedlichsten Phenolen unter effizienter Stromnutzung zur Kreuzkupplung gebracht werden. Reagenzabfälle werden vermieden und die Menge gebildeter Nebenprodukte ist gering. Bei dieser direkten C,C-bzw. C,O-Kupplung wird lediglich Elektrizität verwendet, was zur Abspaltung von Protonen aus den verwendeten Molekülen führt.

In einer Variante des Verfahrens erfolgt die Umsetzung im Verfahrensschritt a) bei einer Temperatur im Bereich von 80 °C bis 120 °C.

In einer bevorzugten Variante des Verfahrens erfolgt die Umsetzung im Verfahrensschritt a) bei einer Temperatur im Bereich von 90 °C bis 110 °C.

In einer Variante des Verfahrens wird das Natriumhydrogensulfit in Verfahrensschritt a) in einer Menge von 2 bis 8 Äquivalenten bezogen auf das Naphthol zugesetzt.

In einer bevorzugten Variante des Verfahrens wird das Natriumhydrogensulfit in Verfahrensschritt a) in einer Menge von 4 bis 6 Äquivalenten bezogen auf das Naphthol zugesetzt.

In einer Variante des Verfahrens weist das Naphthol in Verfahrensschritt a) eine Struktur gemäß der allgemeinen Formel I auf: 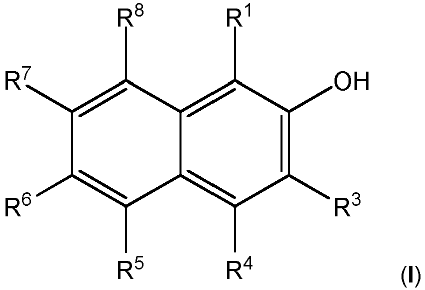 und
R¹, R³, R⁴, R⁵, R⁶, R⁷, R⁸ jeweils unabhängig voneinander ausgewählt sind aus: -H, -(C1-C₁₂)-Alkyl, -O-(C₁-C₁₂)-Alkyl, -(C₃-C₁₂)-Cycloalkyl,
mindestens einer der Reste R¹, R³ für -H steht,
und die Alkyl- und Cycloalkyl-Reste jeweils unabhängig voneinander mit einem oder mehreren Substituenten ausgewählt aus: -(C₁-C₁₂)-Alkyl, -(C₃-C₁₂)-Cycloalkyl, -O-(C₁-C₁₂)-Alkyl, -O-(C₃-C₁₂)-Cycloalkyl, substituiert sein können.

In einer Variante des Verfahrens steht R¹ für -H.

In einer Variante des Verfahrens stehen R¹, R³, R⁴, R⁵, R⁶, R⁷, R⁸ jeweils für-H.

In einer Variante des Verfahrens weist das Amin in Verfahrensschritt a) eine Struktur gemäß der allgemeinen Formel **II** auf: 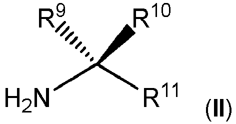 und
R⁹, R¹⁰, R¹¹ jeweils unabhängig voneinander ausgewählt sind aus: -H, -(C₁-C₁₂)-Alkyl, -O-(C₁-C₁₂)-Alkyl, -(C₃-C₁₂)-Cycloalkyl, Phenyl;
und die Alkyl-, Cycloalkyl-, Phenyl-Reste jeweils unabhängig voneinander mit einem oder mehreren Substituenten ausgewählt aus: -(C₁-C₁₂)-Alkyl, -(C₃-C₁₂)-Cycloalkyl, -O-(C₁-C₁₂)-Alkyl, -O-(C₃-C₁₂)-Cycloalkyl, substituiert sein können,
und sich jeder der drei Reste R⁹, R¹⁰, R¹¹ jeweils von den beiden anderen Resten unterscheidet.

In einer Variante des Verfahrens steht R¹¹ für einen Phenylrest steht.

In einer Variante des Verfahrens steht R¹⁰ für -(C₁-C₁₂)-Alkyl steht.

In einer Variante des Verfahrens steht R¹⁰ für -CH₃ steht.

In einer Variante des Verfahrens steht R⁹ für -H steht.

In einer Variante des Verfahrens wird im Verfahrensschritt b) als Anode und Kathode jeweils eine Glaskohlenstoffelektrode verwendet.

In einer Variante des Verfahrens wird als Lösungsmittel im Verfahrensschritt b) 1,1,1,3,3,3-Hexafluorisopropanol verwendet.

In einer Variante des Verfahrens wird als Leitsalz im Verfahrensschritt b) Tributylmethylammoniummethylsulfat verwendet.

In einer Variante des Verfahrens wird das Phenol im Verfahrensschritt b) gegenüber dem Naphthylaminderivat im Überschuss zugesetzt.

In einer bevorzugten Variante des Verfahrens wird das Phenol im Verfahrensschritt b) gegenüber dem Naphthylaminderivat im 2- bis 4-fachen Überschuss zugesetzt.

In einer Variante des Verfahrens weist das Phenol in Verfahrensschritt b) eine Struktur gemäß der allgemeinen Formel **III** auf: 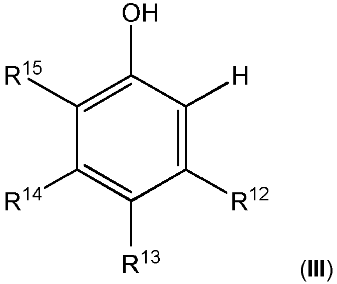 und
R¹², R¹³, R¹⁴, R¹⁵ jeweils unabhängig voneinander ausgewählt sind aus: -H, -(C₁-C₁₂)-Alkyl, -O-(C₁-C₁₂)-Alkyl, -(C₃-C₁₂)-Cycloalkyl, Phenyl;
und die Alkyl-, Cycloalkyl-, Phenyl-Reste jeweils unabhängig voneinander mit einem oder mehreren Substituenten ausgewählt aus: -(C₁-C₁₂)-Alkyl, -(C₃-C₁₂)-Cycloalkyl, -O-(C₁-C₁₂)-Alkyl, -O-(C₃-C₁₂)-Cycloalkyl, substituiert sein können.

In einer Variante des Verfahrens steht R¹³ für -OMe.

In einer Variante des Verfahrens steht R¹⁵ für -OMe.

In einer Variante des Verfahrens steht R¹³ für -Me.

In einer Variante des Verfahrens steht R¹⁵ für -Me.

In einer Variante des Verfahrens steht R¹³ für -tert-Butyl.

In einer Variante des Verfahrens steht R¹⁵ für -tert-Butyl.

In einer Variante des Verfahrens steht R¹⁴ für -H oder -Me.

In einer Variante des Verfahrens steht R¹⁴ für -Me.

Neben dem Verfahren werden auch jene Verbindungen beansprucht, welche eine der folgenden Formel aufweisen:

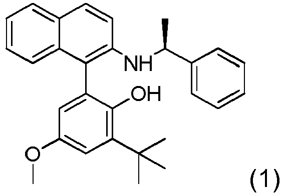

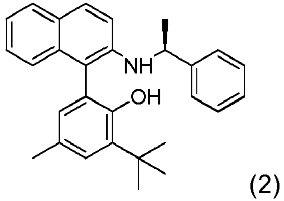

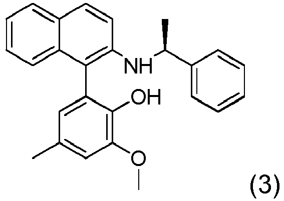

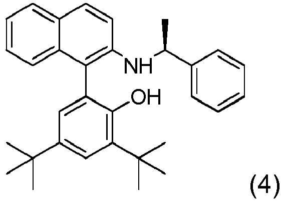

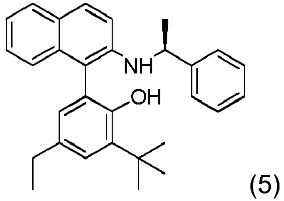

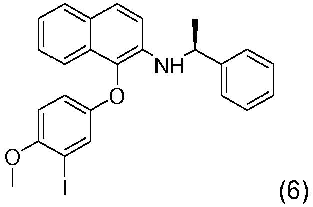

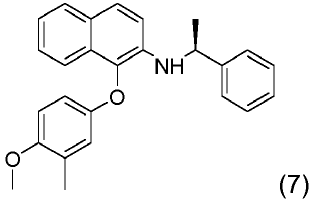

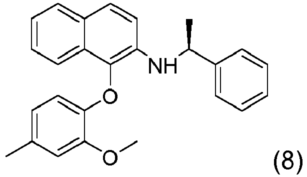

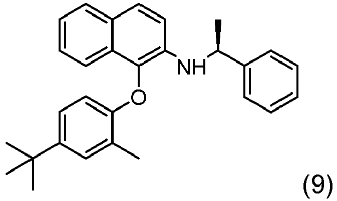

Im Folgenden soll die Erfindung anhand von Ausführungsbeispielen näher erläutert werden.

1. Stufe: Durch Umsetzung von 2-Naphthol mit chiralen Aminen in einer Bucherer-Reaktion können unter einfachen Reaktionsbedingungen ohne die Notwendigkeit von Metallkatalysatoren chirale Naphthylaminderivate erhalten werden.

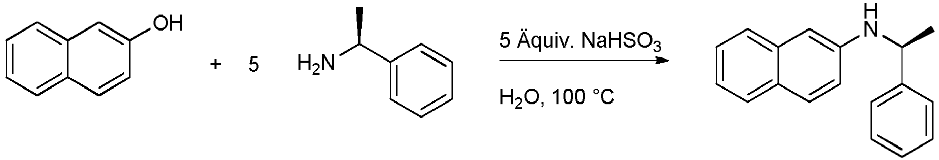

Reaktionsschema 1: Beispiel der Darstellung von (S)-Phenethylaminonaphthalin in einer Bucherer-Reaktion.

2. Stufe: Durch eine anschließende elektrochemische Behandlung mit einer Phenoleinheit kann das Produkt aus der ersten Stufe in ein unsymmetrisches Biaryl bzw. einen Biarylether überführt werden, ohne dass Oxidationsmittel zugegeben, unter Feuchtigkeitsausschluss gearbeitet oder anaerobe Reaktionsführungen eingehalten werden müssen. Durch die Kombination aus Bucherer-Reaktion und direkter C,C- und C,O-Kreuzkupplung wird ein kostengünstiger und umweltschonender Zugang zu diesen Verbindungsklassen eröffnet.

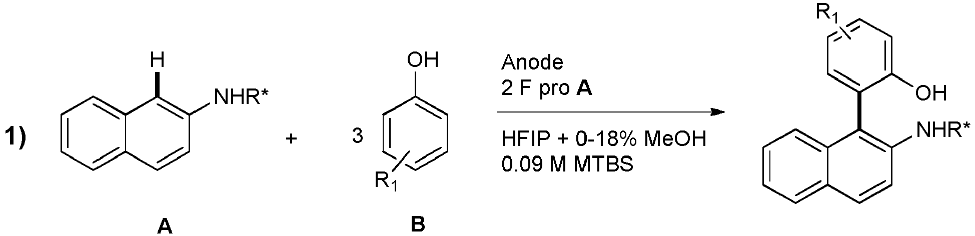

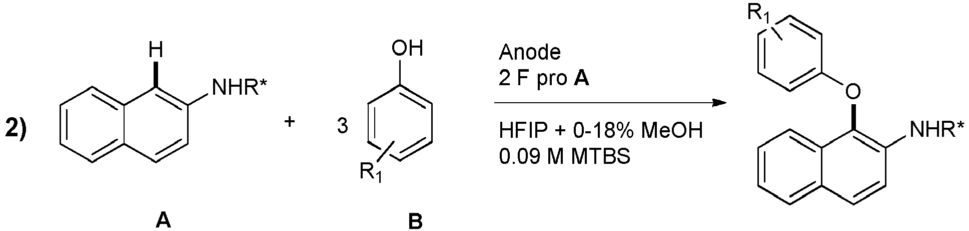

Reaktionsschema 2: Elektrochemische Synthese der unsymmetrischen Biarylverbindungen 1) sowie der Biarylether 2).

Das erfindungsgemäße Verfahren ermöglicht die selektive Oxidation einer chiralen Naphthylaminkomponente **A,** welche zuvor mittels Bucherer-Reaktion dargestellt wurde. Diese ist durch die hohe Reaktivität der gebildeten Radikalspezies in der Lage nukleophil von Komponente **B** angegriffen zu werden. 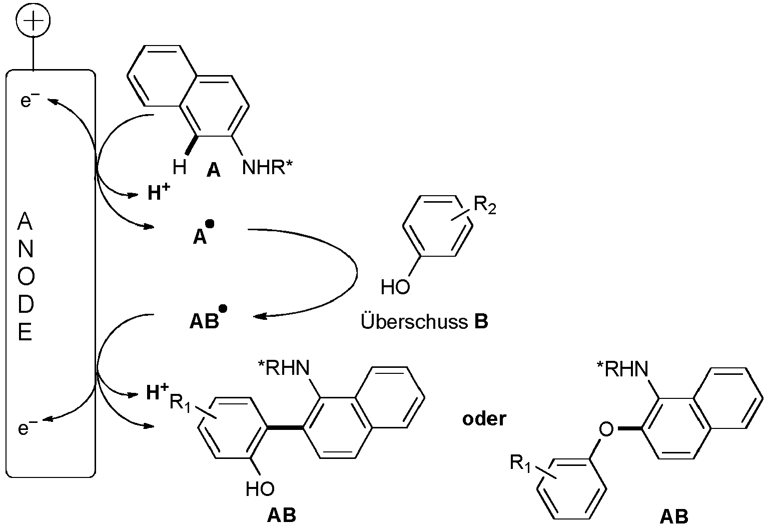 Reaktionsschema 3: schematische Darstellung der elektrochemischen Reaktionen an der Anode

Durch das erfindungsgemäße Verfahren wird eine einfache und kosteneffiziente Methode zur Darstellung chiraler Naphthylaminderivate unter Verwendung der Bucherer-Reaktion etabliert. Die erhaltenen Derivate können im Anschluss mit unterschiedlichsten Phenolen unter effizienter Stromnutzung zur Kreuzkupplung gebracht werden. Reagenzabfälle werden vermieden und die Menge gebildeter Nebenprodukte ist gering. Nicht umgesetzte Edukte können destillativ zurückgewonnen und für weitere Elektrolysen verwendet werden.

Die hier vorgestellten Methoden beschreiben zum ersten Mal den Zugang zu chiralen Naphthylaminderivaten mittels Bucherer-Reaktion, sowie den elektrochemischen Zugang zur gezielten Phenol-Naphthylamin-Kreuzkupplung. Hierdurch bedarf es bei der Synthese keiner Abgangs- oder Schutzgruppenstrategie und die Verwendung von teuren oder giftigen Übergangsmetallkatalysatoren entfällt.

### Experimenteller Teil

| Verbindung | Ausbeute |
|---|---|
| 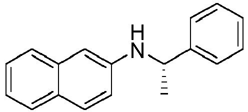 | 70% |
| 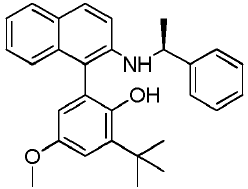 | 48% |
| 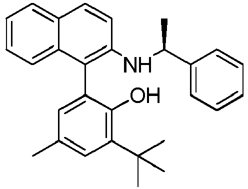 | 32% |
| 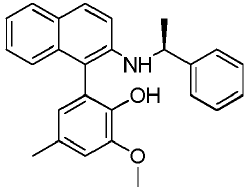 | 26% |
| 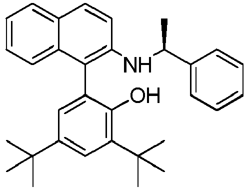 | 40% |
| 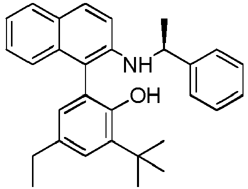 | 25% |
| 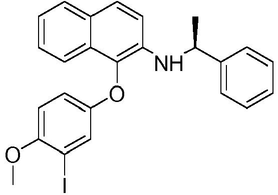 | 8% |
| 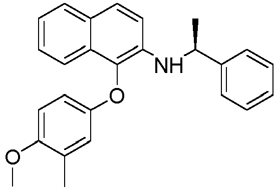 | 12% |
| 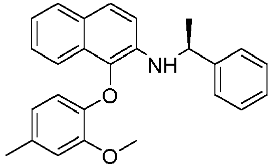 | 23% |
| 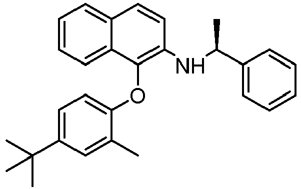 | 21% |

### Allgemeine Methoden

### Chromatographie (GC/GCMS)

Präparative Flüssigchromatographie zur Auftrennung von Stoffgemischen wurde unter Verwendung von Kieselgel 60 M (0.040-0.063 mm) der Firma Machery-Nagel GmbH & CO. KG, Düren durchgeführt. Alle verwendeten Eluenten technischer Qualität (Essigsäureethylester, technische Qualität; Cyclohexan, technische Qualität) wurden vorab destillativ am Rotationsverdampfer gereinigt.

Dünnschichtchromatographie (DC) wurde an PSC-Fertigplatten Kieselgel 60 F254 der Firma Merck KGaA, Darmstadt durchgeführt. Detektion der verschiedenen Substanzen erfolgte zunächst unter UV-Licht und anschließend durch Anfärben mittels Cer-Molybdatophosphorsäure-Reagenz (5.6 g Molybdatophosphorsäure, 2.2 g Cer(IV)-sulfat-Tetrahydrat und 13.3 g konz. Schwefelsäure auf 200 mL Wasser) und anschließendem Erhitzen durch einen Heißluftfön.

### Gaschromatoqraphie (GC/GCMS)

Die gaschromatographischen Untersuchungen (GC) von Produktgemischen und Reinsubstanzen erfolgte mit Hilfe des Gaschromatographen GC-2010 der Firma Shimadzu, Japan. Es wird an einer Quarzkapillarsäule HP-5 der Firma Agilent Technologies, USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min) bzw. ZB5-MSi der Firma Phenomenex, USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: Methode "methode1lang": 100 °C Starttemperatur für 1 min, Heizrate: 15 °C/min, 290 °C Endtemperatur für 30 min) gemessen.

Gaschromatographische Massenspektren (GCMS) von Produktgemischen und Reinsubstanzen wurden mit Hilfe des Gaschromatographen GC-2010 kombiniert mit dem Massendetektor GCMS-QP2010 der Firma Shimadzu, Japan aufgenommen. Es wird an einer Quarzkapillarsäule HP-1 der Firma Agilent Technologies, USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min; GCMS: Temperatur der lonenquelle: 200 °C) oder an einer Quarzkapillarsäule ZB-5 der Firma Phenomenex, USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: "methode1lang" 100 °C Starttemperatur für 1 min, Heizrate: 15 °C/min, 290 °C Endtemperatur für 30 min) gemessen.

### Massenspektrometrie

Alle Elektrosprayionisation-Messungen (ESI+) wurden an einem QTof Ultima 3 der Firma Waters Micromasses, Milford, Massachusetts durchgeführt. EI-Massenspektren sowie die hochaufgelösten EI-Spektren wurden an einem Gerät des Typs MAT 95 XL Sektorfeldgerät der Firma Thermo Finnigan, Bremen, gemessen.

### NMR-Spektroskopie

Die NMR-spektroskopischen Untersuchungen wurden an Multikernresonanzspektrometern des Typs Avance III HD 300 oder Avance II 400 der Firma Bruker, Analytische Messtechnik, Karlsruhe, durchgeführt. Als Lösungsmittel wurde CDCl3 verwendet. Die 1H- und 13C-Spektren wurden gemäß dem Restgehalt an nicht deuteriertem Lösungsmittel nach der NMR Solvent Data Chart der Fa. Cambridge Isotopes Laboratories, USA, kalibriert. Die Zuordnung der 1H- und 13C-Signale erfolgte teilweise mit Hilfe von H,H-COSY, H,H-NOESY, H,C-HSQC und H,C-HMBC-Spektren. Die chemischen Verschiebungen sind als δ-Werte in ppm angegeben. Für die Multiplizitäten der NMR-Signale wurden folgende Abkürzungen verwendet: s (Singulett), bs (breites Singulett), d (Dublett), t (Triplett), q (Quartett), m (Multiplett), dd (Dublett von Dublett), dt (Dublett von Triplett), tq (Triplett von Quartett). Alle Kopplungskonstanten J wurden mit der Anzahl der eingeschlossenen Bindungen in Hertz (Hz) angegeben. Die bei der Signalzuordnung angegebene Nummerierung entspricht der in den Formelschemata angegebenen Bezifferung.

### Allgemeine Arbeitsvorschriften

### AAV1: Elektrochemische Kreuzkupplung in einer Becherglaszelle

3.80 mmol der zu oxidierenden Spezies A (Unterschusskomponente) werden mit einem 2 bis 3-fachen Überschuss (7.6-11.4 mmol) des Kupplungspartners B in 25 mL 1,1,1,3,3,3 Hexafluorisopropanol (HFIP) in einer Becherglaszelle an Glaskohlenstoff Elektroden umgesetzt. Als Leitsalz wird Tributylmethylammoniummethylsulfat (MTBS) mit einer Konzentration von 0.09 M verwendet. Die Elektrolyse erfolgt galvanostatisch. Die Elektrolyse wird bei 22-50 °C durchgeführt. Verdampfendes HFIP wird mit Hilfe eines Dimrothkühlers redestilliert und der Elektrolyse zugeführt. Nach Ende der Elektrolyse wird der Zellinhalt in einen 100 mL Rundhalskolben überführt und das Lösungsmittel unter vermindertem Druck am Rotationsverdampfer bei 50 °C, 70-200 mbar entfernt. Mineralisationsprodukte sowie das enthaltene Leitsalz werden durch Elution mittels Essigsäureethylester: Cyclohexan (1:9 ca. 400 mL) über 50 g Kieselgel 60 abgetrennt. Nicht umgesetztes Edukt wird mittels Kurzwegdestillation zurückerhalten (100 °C, 10-3 mbar). Die entstandenen Reaktionsprodukte werden wie jeweils angegeben säulenchromatographisch getrennt.

### Elektrodenmaterial:

| | |
|---|---|
| Anode: | Glaskohlenstoff |
| Kathode: | Glaskohlenstoff |

### Elektrolysebedingungen:

| | |
|---|---|
| Temperatur: | 22-50 °C |
| Stromdichte: | 2.8 mA/cm2 |
| Ladungsmenge: | 2.0 F bezogen auf die Unterschusskomponente |

### Zweistufige Synthese der Biarylether sowie der Biaryverbindungen

### (S)-(-)-2-α-Methylbenzylaminonaphthalin

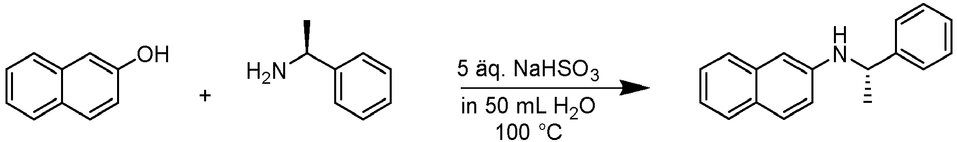

6.0 g (42 mmol, 1.0 Äquiv.) 2-Naphthol werden mit 21.6 g (210 mmol, 5.0 Äquiv.) Natriumhydrogensulfit und 27.0 mL (S)-1-Phenylethylamin (210 mmol, 5.0 Äquiv.) sowie 50 mL Wasser in ein 150 mL Druckrohr gegeben und für 21 h bei 100 °C zur Reaktion gebracht. Die Reaktionslösung wird mit Wasser versetzt und der entstandene Niederschlag abgesaugt. Kristallisation in der Siedehitze aus Cyclohexan (2mL/g) liefert das gewünschte Produkt als leicht gelblichen Feststoff.
Ausbeute: 70% (7.2 g, 29.11 mmol)
Charakterisierung:
   GC (Methode "hart", Säule: HP-5): tR = 15.9 min
   Rf (CH:EE = 9:1): 0.58
   Drehwert (Aceton, 578 nm, c = 3·10-3 g/mL): [α]24: -176°
   Drehwert Literatur (Aceton, 578 nm): [α]24: -178[x]

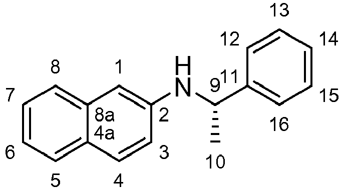

1H-NMR (400 MHz, DMSO-d6) δ[ppm] = 1.46 (d, 3J12-H,11-H = 6.8 Hz, 3H, 10-H), 4.59 (m, 1H, 9-H), 6.49 (d, 3JNH,11-H = 7.1 Hz, 1H, NH), 6.51 (d, 4J1-H,3-H = 2.2 Hz, 1H, 1-H), 7.01-7.06 (m, 2H), 7.15-7.23 (m, 2H), 7.26-7.30 (m, 2H, 13-H, 16-H), 7.36 (m, 1H), 7.41-7.43 (m, 2H, 12-H, 16-H), 7.56 (m, 2H).
13C-NMR (101 MHz, DMSO-d6) δ[ppm] = 24.58 (10-C), 52.00 (9-C), 103.74 (1-C), 118.46 (3-C), 120.98, 125.30, 125.87 (14-C), 125.94 (12-C, 16-C), 126.31, 126.48, 127.33, 128.18 (4-C), 128.33 (13-C, 15-C), 134.81 (8a-C), 145.73 (2-C), 145.80 (11-C).

### 1-(3'-(1,1-Dimethylethyl)-2'-hydroxy-5'-methoxyphenyl)-2-(S)-α-methylbenzylaminonaphthalin

Die Durchführung der Elektrolyse erfolgt gemäß der AAV1 bei 50 °C mit 1.87 g (7.57 mmol, 2.0 Äquiv.) (S)-(-)-2-α-Methylbenzylaminonaphthalin und 680 mg (3.78 mmol, 1.0 Äquiv.) 3-(1,1-Dimethylethyl)-4-hydroxyanisol. Die Stromdichte beträgt 2.8 mA/cm2 und die Ladungsmenge 2.0 F pro 3-(1,1-Dimethylethyl)-4-hydroxyanisol (Q = 731 C). Das Rohprodukt wurde säulenchromatographisch an Kieselgel 60 mit einem Laufmittelgemisch von 9:1 (Cyclohexan:Ethylacetat) vorgereinigt. Die erhaltenen Fraktionen wurden auf Vorhandensein des Produktes geprüft, Edukte durch Kurzwegdestillation (100 °C, 10 -3 mbar) entfernt und der Rückstand erneut an Kieselgel 60 eluiert (Cyclohexan:Ethylacetat = 9:1). Als Produkt wird ein roter, glasartiger Feststoff erhalten. 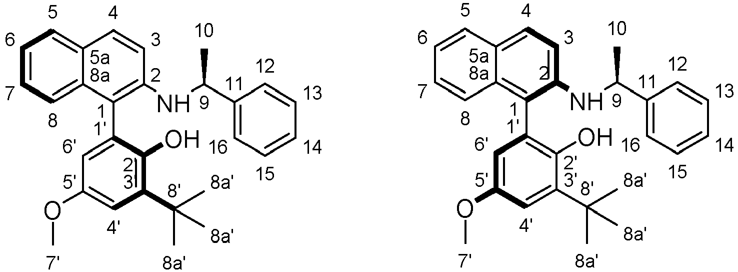
Ausbeute Diastereomerengemisch (1:1): 775 mg (1.82 mmol, 48%)
GC (Methode hart, HP-5): tR = 19.7 min
Rf (Cyclohexan:Ethylacetat = 9:1): 0.52

1H-NMR des Diastereomerengemischs (400 MHz, DMSO-d6) δ [ppm] = 1.29/1.34 (d, 3J10-H,9-H = 6.6/6.7 Hz, 3H, 10-H), 1.43/1.44 (s, 9H, 8a'-H), 3.68/3.69 (s, 3H, 7'-H), 4.25/4.45 (d, 3JNH,9-H =7.0/7.7 Hz, NH, 1H), 4.65/4.74 (m, 1H, 9-H), 6.45/6.47 (d, 4J6'-H,4'-H = 3.1/3.1 Hz, 1H, 6'-H), 6.89 (m, 1H, 4'-H), 6.96/7.00 (d, 3J3-H,4-H = 9.0/9.1 Hz, 1H, 3-H), 7.13-7.20 (m, 3H), 7.25-7.31 (m, 4H, 12-H, 16-H, 13-H, 15-H), 7.41 (d, J = 7.2 Hz, 1H), 7.43/7.57(s, 1H), 7.65-7.70 (m, 2H, 4-H).
13C-NMR des Diastereomerengemischs (101 MHz, DMSO-d6) δ[ppm] = 24.54/24.88 (10-C), 29.42/29.44 (8a'-C), 34.87/34.91 (8'-C), 52.15/52.75 (9-C), 55.16/55.18 (7'-C), 112.90, 113.16/113.48 (6'-C), 113.64, 115.23, 115.38, 115.58, 121.40, 123.70, 123.85, 123.88, 124.70, 125.77/125.90 (12-C, 16-C), 125.94, 126.05, 126.63, 126.73, 127.07, 127.10, 127.81, 128.33, 128.38/128.46 (13-C, 15-C), 128.63/128.71 (4-C), 133.53/133.61 (2-C), 138.80/139.20 (3'-C), 142.18, 142.88, 145.36/145.51 (11-C), 147.13/147.17 (2'-C), 152.38/152.47 (5'-C).

| | | |
|---|---|---|
| HRMS (ESI, pos. mode): m/z für C29H31 NO2 [M+H+]: | berechnet: | 426.2428 |
| | gefunden: | 426.2431 |

### 1-(3'-(1,1-Dimethylethyl)-2'-hydroxy-5'-methylphenyl)-2-(S)-α-methylbenzylaminonaphthalin

Die Elektrolyse wird gemäß AAV1 bei 50 °C mit 1.87 g (11.36 mmol, 3.0 Äquiv.) 2-(1,1-Dimethylethyl)-4-methylphenol und 936 mg (3.80 mmol, 1.0 Äquiv.) (S)-(-)-2-α-Methylbenzylaminonaphthalin durchgeführt. Die Stromdichte beträgt 2.8 mA/cm2und die Ladungsmenge 2.0 F pro (S)-(-)-2-α-Methylbenzylaminonaphthalin (Q = 731 C). Nach Abtrennung des Lösungsmittels wird die Produktmischung säulenchromatographisch an Kieselgel 60 mit einem Eluenten von 9:1 (Cyclohexan:Ethylacetat) vorgereinigt. Die Fraktionen werden auf Produkt geprüft und enthaltene Edukte durch Kurzwegdestillation entfernt (100 °C, 10-3 mbar). Eine weitere säulenchromatographische Aufreinigung an Kieselgel 60 mit einem Laufmittel-Gradienten von 100:00 auf 70:30 (Cyclohexan:Dichlormethan) liefert das gewünschte Produkt als leicht gelblichen Feststoff. 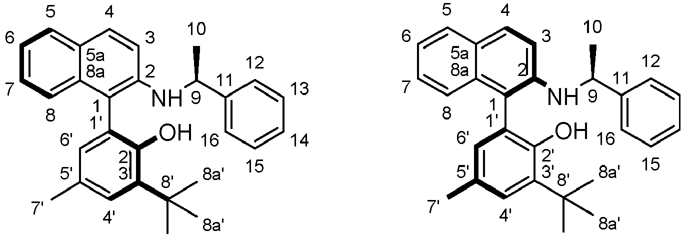
Ausbeute Diastereomerengemisch (1:1): 500 mg (1.20 mmol, 32%)
GC (Methode "methode1lang", ZB-5): tR = 21.0 min
Rf (Cyclohexan:Ethylacetat = 9:1): 0.71

1H-NMR des Diastereomerengemischs (400 MHz, DMSO-d6) δ [ppm] = 1.27/1.33 (d, 3J10-H,9-H = 6.7/6.7 Hz, 3H, 10-H), 1.44/1.44 (s, 9H, 8a'-H), 2.27/2.28 (s, 3H, 7'-H), 4.16/4.36 (d, 3JNH,9-H = 7.0/7.6 Hz, 1H, NH), 4.65/4.73 (m, 1H, 9-H), 6.67/6.72 (dd, 4;4J6'-H,4'-H;6'-H,7'-H = 2.3, 0.8/ 2.2/0.9, 1H, 6'-H), 6.96/7.00 (d, 3J3-H,4-H = 9.0/9.0 Hz, 1H, 3-H), 7.12-7.30 (m, 8H), 7.39-7.42 (m, 1H), 7.60/7.72 (s, 1H, OH), 7.64-7.70 (m, 2H).
13C-NMR des Diastereomerengemischs (101 MHz, DMSO-d6) δ [ppm] = 20.58/20.60 (7'-C), 24.48/24.84 (10-C), 29.55/29.55 (8a'-C), 34.65/34.68 (8'-C), 52.10/52.91 (9-C), 115.19, 115.28, 115.35, 115.45, 121.34, 121.38, 123.05, 123.75, 123.77, 123.89, 125.74, 125.86, 125.96, 126.62, 126.73, 127.08, 127.16, 127.20, 127.79, 128.13, 128.27, 128.37, 128.45, 128.58, 128.64, 130.07/130.23 (5'-C), 133.64/133.76, 136.94/137.28 (3'-C), 142.27/143.02, 145.33/145.43 (11-C), 151.01/151.11 (2'-C).

| | |
|---|---|
| HRMS (ESI, pos. mode): m/z für C29H31NO (M+H+): | berechnet: 410.2478 |
| | gefunden: 410.2472 |

### 1-(2'-Hydroxy-3'-methoxy-5'-methylphenyl)-2-(S)-α-methylbenzylaminonaphthalin

Die Elektrolyse wird gemäß AAV1 bei 50 °C mit 1.57 g (11.36 mmol, 3.0 Äquiv.) 4-Methylguajakol und 936 mg (3.80 mmol, 1.0 Äquiv.) (S)-(-)-2-α-Methylbenzylaminonaphthalin durchgeführt. Die Stromdichte beträgt 2.8 mA/cm2 und die Ladungsmenge 2.0 F pro (S)-(-)-2-α-Methylbenzylaminonaphthalin (Q = 731 C). Nach Abtrennung des Lösungsmittels wird die Produktmischung säulenchromatographisch an Kieselgel 60 mit einem Eluenten von 9:1 (Cyclohexan:Ethylacetat) vorgereinigt. Die Fraktionen werden auf Produkt geprüft und enthaltene Edukte durch Kurzwegdestillation entfernt (100 °C, 10-3 mbar). Eine weitere säulenchromatographische Aufreinigung an Kieselgel 60 mit einem Laufmittel-Gradienten von 100:00 auf 70:30 (Cyclohexan:Dichlormethan) liefert das gewünschte Produkt als roten Feststoff. 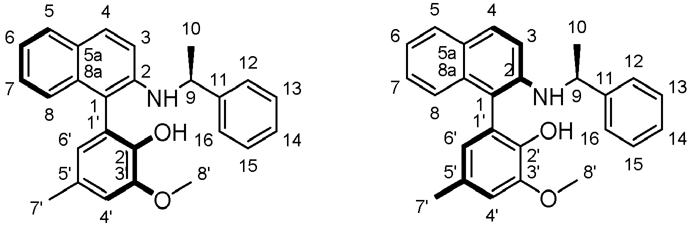
Ausbeute Diastereomerengemisch (1:1): 384 mg (1.00 mmol, 26%)
GC (Methode "methode1lang", ZB-5): tR = 22.3 min
Rf (Cyclohexan:Ethylacetat = 9:1): 0.23

1H-NMR (400 MHz, CDCl3) δ [ppm] = 1.30/1.33 (d, 3J10-H,9-H = 6.7/6.7 Hz, 3H, 10-H), 2.31/2.31 (s, 3H, 7'-H), 3.90/3.89 (s, 3H, 8'-H), 4.24/4.14 (d, 3JNH,9-H = 7.7/7.5 Hz, 1H, NH), 4.73/4.73 (m, 1H, 9-H), 6.49/6.40 (dd, 4;4J6'-H,4'-H; 6'-H,7'-H = 1.9, 0.9/2.0, 1.0,1H, 6'-H), 6.88/7.02 (d, J3-H,4-H = 9.0/8.8 Hz,1H, 3-H), 6.93/6.93 (d, 4J4'-H, 6'-H = 2.0/1.9 Hz, 1H, 4'-H), 7.04-7.13 (m, 2H), 7.14-7.21 (m, 2H), 7.22-7.30 (m, 2H), 7.36-7.41 (m, 2H), 7.58/ 7.62 (d, 3J4-H,3-H = 8.9/9.0 Hz, 1H, 4-H), 7.63-7.65 (m, 1H) 8.39/8.26 (s, 1H, OH).
13C-NMR (100 MHz, DMSO-d6) δ [ppm] = 20.73/20.79 (7'-C), 25.08/24.49 (10-C), 52.12/52.72 (9-C), 55.65/55.69 (8'-C), 112.12/112.20 (4'-C), 114.83/115.30 (3-C), 116.34, 116.76, 121.10, 121.28, 122.40, 122.32, 123.57, 123.90, 123.96, 125.64, 125.69, 125.81, 125.92, 126.47, 126.49, 126.67, 126.69, 127.58, 127.58, 127.76, 127.84, 128.31, 128.41, 128.44, 128.47, 128.47, 133.14, 133.04, 141.92, 142.22, 142.39, 142.41 (2'-C), 145.53/145.49 (11-C), 148.00/148.00 (3'-C).

| | |
|---|---|
| HRMS (ESI, pos. mode): m/z für C26H25NO2 (M+Na+): | berechnet: 406.1778 |
| | gefunden: 406.1785 |

### 1-(3'-(1,1-Dimethylethyl)-5'-(1,1-dimethylethyl)-2'-hydroxyphenyl)-2-(S)-α-methylbenzylaminonaphthalin

Die Elektrolyse wird gemäß AAV1 bei Raumtemperatur mit 2.34 g (11.36 mmol, 3.0 Äquiv.) 2,4-Bis-(1,1-dimethylethyl)phenol und 936 mg (3.80 mmol, 1.0 Äquiv.) (S)-(-)-2-α-Methylbenzylaminonaphthalin durchgeführt. Die Stromdichte beträgt 2.8 mA/cm2 und die Ladungsmenge 2.0 F pro (S)-(-)-2-α-Methylbenzylaminonaphthalin (Q = 731 C). Nach Abtrennung des Lösungsmittels wird die Produktmischung säulenchromatographisch an Kieselgel 60 mit einem Eluenten von 9:1 (Cyclohexan:Ethylacetat) vorgereinigt. Die Fraktionen werden auf Produkt geprüft und enthaltene Edukte durch Kurzwegdestillation entfernt (100 °C, 10-3 mbar). Eine weitere säulenchromatographische Aufreinigung an Kieselgel 60 mit einem Laufmittel-Gradienten von 100:00 auf 70:30 (Cyclohexan:Dichlormethan) liefert das gewünschte Produkt als leicht gelblichen Feststoff. 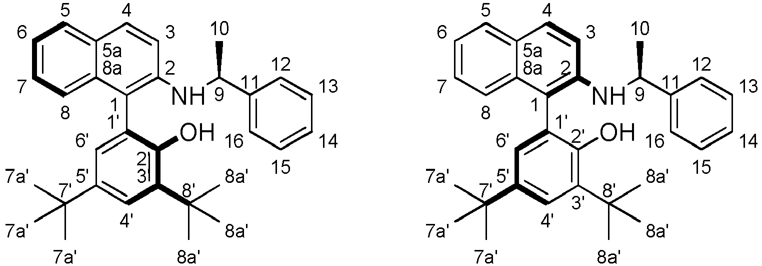
Ausbeute Diastereomerengemisch (1:1): 693 mg (1.50 mmol, 40%)
GC (Methode "methode1lang", ZB-5): tR = 22.3 min
Rf (Cyclohexan:Ethylacetat = 9:1): 0.68

1H-NMR des Diastereomerengemischs (400 MHz, DMSO-d6) δ [ppm] = 1.25-1.31 (m, 12H, 10-H, 7a'-H), 1.46/1.46 (s, 9H, 8a'-H), 4.19/4.42 (d, 3JNH,9-H = 7.7/7.7 Hz, 1H, NH), 4.62/4.77 (m, 1H, 9-H), 6.90/6.93 (d, 4J6'-H,4'-H = 2.44/2.44 Hz, 1H, 6'-H), 7.00/7.04 (d, 3J3-H,4-H = 9.0/9.0 Hz, 1H, 3-H), 7.10-7.30 (m, 7H), 7.33-7.39 (m, 2H), 7.60/7.72 (s, 1H, OH), 7.64-7.71 (m, 2H).
13C-NMR des Diastereomerengemischs (101 MHz, DMSO-d6) δ [ppm] = 24.71/24.71 (10-C), 29.61/29.64 (8a'-C), 31.51/31.51 (7a'-C), 34.02/34.02 (7'-C), 34.93/34.95 (8'-C), 52.38/52.46 (9-C), 115.48, 115.61, 115.75, 116.16, 121.38, 121,47, 122.56, 122.76, 122.91, 123.29, 123.67, 123.86, 125.70, 125.88, 126.03, 126.29, 126.61, 126.68, 126.75, 127.15, 127.25, 127.84, 127.85, 128.38, 128.41, 128.60, 128.63, 133.59, 133.63, 136.26/136.61 (3'-C), 141.46/141.52 (5'-C), 142.57/142.98, 145.27/145.49 (11-C), 151.11/151.11 (2'-C).

| | |
|---|---|
| HRMS (ESI, pos. mode): m/z für C32H37NO (M+H+): | berechnet: 452.2948 |
| | gefunden: 452.2948 |

### 1-(3'-(1,1-Dimethylethyl)-5'-ethyl-2'-hydroxyphenyl)-2-(S)-α-methylbenzylaminonaphthalin

Die Elektrolyse wird gemäß AAV1 bei Raumtemperatur mit 2.02 g (11.36 mmol, 3.0 Äquiv.) 2-(1,1-Dimethylethyl)-4-ethylphenol und 936 mg (3.80 mmol, 1.0 Äquiv.) (S)-(-)-2-α-Methylbenzylaminonaphthalin durchgeführt. Die Stromdichte beträgt 2.8 mA/cm2 und die Ladungsmenge 2.0 F pro (S)-(-)-2-α-Methylbenzylaminonaphthalin (Q = 731 C). Nach Abtrennung des Lösungsmittels wird die Produktmischung säulenchromatographisch an Kieselgel 60 mit einem Eluenten von 9:1 (Cyclohexan:Ethylacetat) vorgereinigt. Die Fraktionen werden auf Produkt geprüft und enthaltene Edukte durch Kurzwegdestillation entfernt (100 °C, 10-3 mbar). Eine weitere säulenchromatographische Aufreinigung an Kieselgel 60 mit einem Laufmittel-Gradienten von 100:00 auf 70:30 (Cyclohexan:Dichlormethan) liefert das gewünschte Produkt als leicht gelblichen Feststoff. 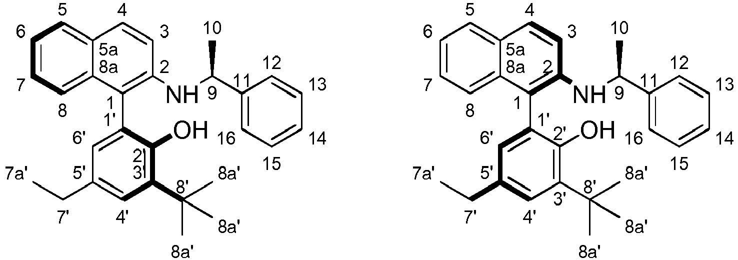
Ausbeute Diastereomerengemisch (70:30): 407 mg (0.90 mmol, 25%)
GC (Methode "methode1lang", ZB-5): tR = 18.7 min
Rf (Cyclohexan:Ethylacetat = 9:1): 0.71

1H-NMR des Diastereomerengemischs (400 MHz, DMSO-d6) δ [ppm] = 1.16-1.22 (m, 3H, 7a'-H), 1.27/1.32 (d, 3J10-H,9-H = 6.6/6.6 Hz, 3H, 10-H), 1.45/1.45 (s, 9H, 8a'), 2.55-2.63 (m, 2H, 7'-H), 4.17/4.38 (d, 3JNH,9-H = 7.3/7.6 Hz, 1H, NH), 4.62/4.77 (p, J = 6.8 Hz, 1H, 9-H), 6.71/6.75 (d, 4J6'-H, 4'-H = 2.2/2.1 Hz, 1H, 6'-H), 6.98/7.02 (d, 3J3-H,4-H = 9.0/9.0 Hz, 1H, 3-H), 7.11-7.41 (m, 9H), 7.60/7.70 (s, 1H, OH), 7.64-7.69 (m, 2H).
13C-NMR des Diastereomerengemischs (101 MHz, DMSO-d6) δ [ppm] = 15.76/15.87 (7a'-C), 24.54/24.78 (10-C), 27.63/27.66 (7'-C), 29.54/29.52 (8a'-C), 34.70/34.73 (7'-C), 52.21/52.75 (9-C), 115.36, 115.43, 115.48, 115.62, 121.37, 123.72, 123.87, 125.73, 125.86, 125.98, 126.01, 126.64, 126.73, 127.13, 127.16, 127.80, 128.37, 128.42, 128.55, 128.58, 128.63, 128.81, 133.62, 133.69, 134.58, 134.58, 134.47, 136.89, 137.25, 142.38, 143.02, 145.31, 145.45, 151.25, 151.29.

| | |
|---|---|
| HRMS (ESI, pos. mode): m/z für C30H30NO (M+H+): | berechnet: 424.2635 |
| | gefunden: 424.2633 |

### 1-(3'-Iod-4'-methoxyphenoxy)-2-(S)-α-methylbenzylaminonaphthalin

Die Elektrolyse wird gemäß AAV1 bei 50 °C mit 2.84 g (11.36 mmol, 3.0 Äquiv.) 3-lod-4-methoxyphenol und 936 mg (3.80 mmol, 1.0 Äquiv.) (S)-(-)-2-α-Methylbenzylaminonaphthalin durchgeführt. Die Stromdichte beträgt 2.8 mA/cm2 und die Ladungsmenge 2.0 F pro (S)-(-)-2-α-Methylbenzylaminonaphthalin (Q = 731 C). Nach Abtrennung des Lösungsmittels wird die Produktmischung säulenchromatographisch an Kieselgel 60 mit einem Eluenten von 9:1 (Cyclohexan:Ethylacetat) vorgereinigt. Die Fraktionen werden auf Produkt geprüft und enthaltene Edukte durch Kurzwegdestillation entfernt (100 °C, 10-3 mbar). Eine weitere säulenchromatographische Aufreinigung an Kieselgel 60 mit einem Laufmittel-Gradienten von 100:00 auf 70:30 (Cyclohexan:Dichlormethan) liefert das gewünschte Produkt als leicht gelblichen Feststoff. 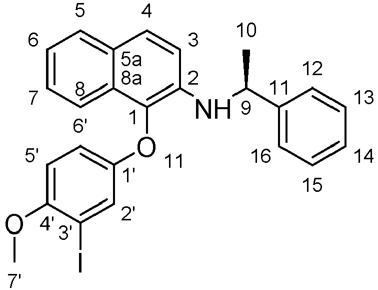
Ausbeute: 155 mg (0.30 mmol, 8%)
GC (Methode "methode1lang", ZB-5): tR = 24.5 min
Rf (Cyclohexan:Ethylacetat = 9:1): 0.46

1H-NMR (400 MHz, DMSO-d6) δ [ppm] = 1.43 (d,3J10-H,9-H= 6.7 Hz, 3H, 10-H), 3.77 (s, 3H, 7'-H), 4.80 (m, 1H, 9-H), 5.60 (d, 3JNH,9-H = 7.9 Hz, 1H, NH), 6.87 (dd, 3;4J6'-H,5'-H;6'-H,2'-H = 9.0, 2.9 Hz, 1H, 6'-H), 6.95 (d, 3J5'-H,6'-H = 9.0 Hz, 1H, 5'-H), 7.09 (d, 3J3-H,4-H = 9.0 Hz, 1H, 3-H), 7.13-7.19 (m, 2H), 7.21 (d, 4J2'-H,6'-H = 2.9 Hz, 1H, 2'-H), 7.24-7.36 (m, 5H), 7.46 (dd, J = 8.4, 1.1 Hz, 1H), 7.56 (d, 3J4-H,3-H = 9.0 Hz, 1H, 4-H), 7.70 (dd, J = 8.2, 1.0 Hz, 1H).
13C-NMR (101 MHz, DMSO-d6) δ [ppm] = 24.43 (C-10), 51.98 (9-C), 56.74 (7'-C), 86.21 (3'-C), 112,13 (5'-C), 115.91 (3-C), 116.04 (6'-C), 119.17 (5-C oder 8-C), 121.92 (6-C oder 7-C), 125.21 (2'-C), 125.86 (Phenyl-C), 125.95 (4-C), 126.61 (Phenyl-C), 126.70 (6-C oder 7-C), 127.61, 127.93, 128.41, 128.73 (5-C oder 8-C), 131.85(1-C), 137.60, 145.73 (11-C), 152.20 (1'-C), 153.04 (4'-C).

| | |
|---|---|
| HRMS (ESI, pos. mode): m/z für C25H22INO (M+H+): | berechnet: 496.0768 |
| | gefunden: 496.0762 |

### 1-(4'-Methoxy-3'-methylphenoxy)-2-(S)-α-methylbenzylaminonaphthalin

Die Elektrolyse wird gemäß AAV1 bei 50 °C mit 1.57 g (11.36 mmol, 3.0 Äquiv.) 3-Methyl-4-methoxyphenol und 936 mg (3.80 mmol, 1.0 Äquiv.) (S)-(-)-2-α-Methylbenzylaminonaphthalin durchgeführt. Die Stromdichte beträgt 2.8 mA/cm2 und die Ladungsmenge 2.0 F pro (S)-(-)-2-α-Methylbenzylaminonaphthalin (Q = 731 C). Nach Abtrennung des Lösungsmittels wird die Produktmischung säulenchromatographisch an Kieselgel 60 mit einem Eluenten von 9:1 (Cyclohexan:Ethylacetat) vorgereinigt. Die Fraktionen werden auf Produkt geprüft und enthaltene Edukte durch Kurzwegdestillation entfernt (100 °C, 10-3 mbar). Eine weitere säulenchromatographische Aufreinigung an Kieselgel 60 mit einem Laufmittel-Gradienten von 100:00 auf 70:30 (Cyclohexan:Dichlormethan) liefert das gewünschte Produkt als leicht rötlichen Feststoff. 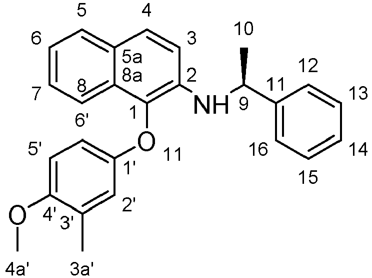
Ausbeute: 170 mg (0.40 mmol, 12%)
GC (Methode "methode1lang", ZB-5): tR = 24.5 min
Rf (Cyclohexan:Ethylacetat = 9:1): 0.51

1H-NMR (400 MHz, DMSO-d6) δ [ppm] = 1.41 (d,3J10-H,9-H = 6.7 Hz, 3H, 10-H), 2.09 (s, 3H, 3a'-H), 3.72 (s, 3H, 4a'-H), 4.80 (m, 1H, 9-H), 5.35 (d, 3JNH,9-H = 8.1 Hz, 1H, NH), 6.55 (dd, 3;4J 6'-H,2'-H;6'-H,5'-H = 8.9, 3.0 Hz, 1H, 6'-H), 6.73 (dd, 4;4J2'-H,6'-H, 2'-H,3a'-H = 3.1, 0.88 Hz, 1H, 2'-H), 6.82 (d, 3J5'-H,6'-H = 8.9, 5'-H), 7.10 (d, 3J3-H,4-H = 9.0 Hz, 3-H), 7.12-7.18 (m, 2H), 7.22-7.34 (m, 5H), 7.48 (dd, J = 8.5, 1.1 Hz, 1H), 7.54 (d, 3J4-H,3-H = 9.0 Hz, 1H, 4-H), 7.70 (dd, J = 8.1, 1.0 Hz, 1H).
13C-NMR (101 MHz, DMSO-d6) δ [ppm] = 16.70 (3a'-C), 24.91 (10-C), 52.46 (9-C), 56.02 (4a'-C), 111.57(5'-C), 112.93 (6'-C), 116.39 (3-C), 117.88 (2'-C), 119.94 (5-C, 8-C), 122.30, 126.01 (4-C), 126.34, 126.81, 127.09, 127.29, 128.32, 128.79, 132.83, 138.07, 146.12, 151,79, 152.77 (4'-C).

| | |
|---|---|
| HRMS (ESI, pos. mode): m/z für C26H25NO2 (M+H+): | berechnet: 384.1958 |
| | gefunden: 384.1968 |

### 1-(2'-Methoxy-4'-methylphenoxy)-2-(S)-(-)-α-methylbenzylaminonaphthalin

Die Elektrolyse wird gemäß AAV1 bei 50 °C mit 1.57 g (11.36 mmol, 3.0 Äquiv.) 3-Methyl-4-methoxyphenol und 936 mg (3.80 mmol, 1.0 Äquiv.) (S)-(-)-2-α-Methylbenzylaminonaphthalin durchgeführt. Die Stromdichte beträgt 2.8 mA/cm2 und die Ladungsmenge 2.0 F pro (S)-(-)-2-α-Methylbenzylaminonaphthalin (Q = 731 C). Nach Abtrennung des Lösungsmittels wird die Produktmischung säulenchromatographisch an Kieselgel 60 mit einem Eluenten von 9:1 (Cyclohexan:Ethylacetat) vorgereinigt. Die Fraktionen werden auf Produkt geprüft und enthaltene Edukte durch Kurzwegdestillation entfernt (100 °C, 10-3 mbar). Eine weitere säulenchromatographische Aufreinigung an Kieselgel 60 mit einem Laufmittel-Gradienten von 100:00 auf 70:30 (Cyclohexan:Dichlormethan) liefert das gewünschte Produkt als leicht rötlichen Feststoff. 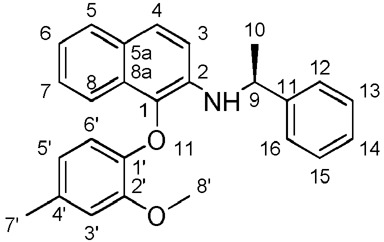
Ausbeute: 340 mg (0.88 mmol, 23%)
GC (Methode hart, HP-5): tR = 18.5 min
Rf (Cyclohexan:Ethylacetat = 9:1) = 0.38

1H-NMR (400 MHz, DMSO-d6) δ[ppm] = 1.40 (d, 3J10-H,9-H = 7.4 Hz, 3H, 10-H), 2.26 (s, 3H, 8'-H), 3.97 (s, 3H, 7'-H), 4.76(m, 1H, 9-H), 5.35 (d, 3JNH,9-H = 8.0 Hz, 1H, NH), 6.20 (d, 3J6'-H,5'-H = 8.1 Hz, 1H, 6'-H), 6.51 (ddd, 3;4;4J5'-H,6'-H,5'-H,3'-H,5'-H,7'-H = 8.2, 2.0, 0.9 Hz , 1H, 5'-H), 7.01 (d, 4J3'-H,5'-H = 2.1 Hz, 1H, 3'-H), 7.06 (d, 3J3-H,4-H = 9.0 Hz, 1H, 3-H), 7.13-7.18 (m, 2H), 7.21-7.27 (m, 4H, 12-H,13-H,15-H,16-H), 7.30-7.34 (m, 1H), 7.52-7.55 (m, 2H, 4-H), 7.69 (d, J=8.0 Hz, 1H).
13C-NMR (100 MHz, DMSO-d6) δ[ppm] = 20.70 (8'-C), 24.45 (10-C), 51.88 (9-C), 55.70 (7'-C), 113.39 (3'-C), 114.97, 115.79 (6'-C), 119.31, 120.74, 121.89 (5'-C), 125.46, 125.82 (12-C, 16-C), 126.51, 126.65, 126.69, 127.79, 127.97, 128.34 (13-C, C-15), 131.89(4'-C), 133.67(1-C), 137.38 (2-C), 144.69(1'-C), 145.51(11-C), 148.64(2'-C).

| | |
|---|---|
| HRMS (ESI, pos. mode): m/z für C26H25NO2 (M+Na+): | berechnet: 406.1778 |
| | gefunden: 406.1779 |

### 1-(4'-(1,1-Dimethylethyl)-2'-methylphenoxy)-2-(S)-α-methylbenzylaminonaphthalin

Die Elektrolyse wird gemäß AAV1 bei 50 °C mit 1.57 g (11.36 mmol, 3.0 Äquiv.) 3-Methyl-4-methoxyphenol und 936 mg (3.80 mmol, 1.0 Äquiv.) (S)-(-)-2-α-Methylbenzylaminonaphthalin durchgeführt. Die Stromdichte beträgt 2.8 mA/cm2, die Ladungsmenge 2.0 F pro (S)-(-)-2-α-Methylbenzylaminonaphthalin (Q = 731 C). Nach Abtrennung des Lösungsmittels wird die Produktmischung säulenchromatographisch an Kieselgel 60 mit einem Eluenten von 9:1 (Cyclohexan:Ethylacetat) vorgereinigt. Die Fraktionen werden auf Produkt geprüft und enthaltene Edukte durch Kurzwegdestillation entfernt (100 °C, 10-3 mbar). Eine weitere säulenchromatographische Aufreinigung an Kieselgel 60 mit einem Laufmittel-Gradienten von 100:00 auf 70:30 (Cyclohexan:Dichlormethan) liefert das gewünschte Produkt als leicht rötlichen Feststoff. 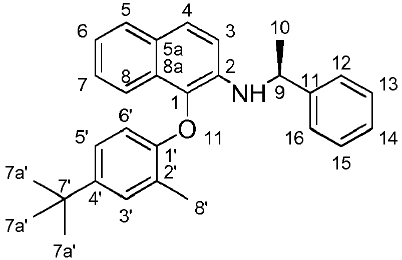
Ausbeute: 320 mg (0.78 mmol, 21%)
GC (Methode "methode1lang", ZB-5): tR = 21.8 min
Rf (Cyclohexan:Ethylacetat = 9:1) = 0.80

1H-NMR (400 MHz, DMSO-d6) δ[ppm] = 1.24 (s, 9H, 7a'-H), 1.42 (d, 3J10-H,9-H = 6.7 Hz, 3H, 10-H), 2.57 (s, 3H, 8'-H), 4.81 (m, 1H, 9-H), 5.36 (d, 3JNH,9-H = 8.0 Hz, 1H, NH), 6.04 (d, 3J6'-H,5'-H = 8.5 Hz, 1H, 6'-H), 6.94 (dd, 3;4J5'-H,6'-H; 5'-H;3'-H = 8.6, 2.3 Hz, 1H, 5'-H), 7.10-7.18 (m, 3H), 7.23-7.37 (m, 7H), 7.55 (d, 3J4-H,3-H = 9.0 Hz, 1H, 4-H), 7.70 (d, J = 8.3 Hz, 1H).
13C-NMR (101 MHz, DMSO-d6) δ[ppm] = 16.62 (8'-C), 24.43 (10-C), 31.37 (7a'-C), 33.78 (7'-C), 52.12 (9-C), 111.98 (6'-C), 115.94, 119.29, 121.87, 123.34 (5'-C), 124.86, 125.55, 125.87, 126.48, 126.63, 126.88, 127.62, 127.85, 128.06, 128.32, 123.29, 137.64 (2-C), 143.60 (4'-C), 145.67 (11-C), 153.68 (1'-C).

| | |
|---|---|
| HRMS (ESI, pos. mode): m/z für C29H31 NO (M+H+): | berechnet: 410.2478 |
| | gefunden: 410.2475 |

Die Durchgeführten Versuche zeigen, dass die Aufgabe durch das erfindungsgemäße Verfahren gelöst wird.

## Patentansprüche

1. Verfahren umfassend die Verfahrensschritte:
a) Umsetzung eines Naphthols, welches in ortho-Position zur OH-Gruppe einen Wasserstoff aufweist, mit einem chiralen primären Amin in Gegenwart von Wasser und unter Zusatz von Natriumhydrogensulfit, so dass ein sekundäres Naphthylaminderivate entsteht,
b) elektrochemische Kupplung des sekundären Naphthylaminderivats aus a) unter Verwendung eines Lösungsmittels und eines Leitsalzes mit einem Phenol, welches in ortho-Position zur OH-Gruppe einen Wasserstoff aufweist, so dass
- entweder eine direkte Kupplung des Phenylrings des Phenols in ortho-Position zur OH-Gruppe mit dem Naphthylringsystem in ortho-Positon zur Aminogruppe erfolgt, oder
- der Sauerstoff des Phenols an das Naphthylringsystem in ortho-Positon zur Aminogruppe gekuppelt wird.

2. Verfahren nach Anspruch 1,
wobei die Umsetzung im Verfahrensschritt a) bei einer Temperatur im Bereich von 80 °C bis 120 °C erfolgt.

3. Verfahren nach Anspruch 1 oder 2,
wobei das Natriumhydrogensulfit in Verfahrensschritt a) in einer Menge von 2 bis 8 Äquivalenten bezogen auf das Naphthol zugesetzt wird.

4. Verfahren nach einem der Ansprüche 1 bis 3,
wobei das Naphthol in Verfahrensschritt a) eine Struktur gemäß der allgemeinen Formel I aufweist: 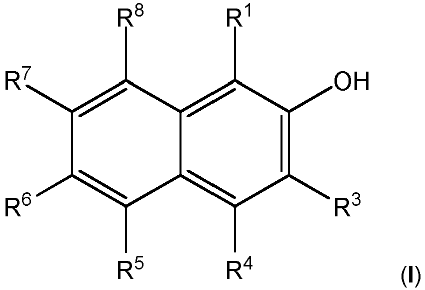 und
R¹, R³, R⁴, R⁵, R⁶, R⁷, R⁸ jeweils unabhängig voneinander ausgewählt sind aus: -H,-(C₁-C₁₂)-Alkyl, -O-(C₁-C₁₂)-Alkyl, -(C₃-C₁₂)-Cycloalkyl,
mindestens einer der Reste R¹, R³ für -H steht,
und die Alkyl- und Cycloalkyl-Reste jeweils unabhängig voneinander mit einem oder mehreren Substituenten ausgewählt aus: -(C₁-C₁₂)-Alkyl, -(C₃-C₁₂)-Cycloalkyl, -O-(C₁-C₁₂)-Alkyl, -O-(C₃-C₁₂)-Cycloalkyl, substituiert sein können.

5. Verfahren nach einem der Ansprüche 1 bis 4,
wobei R¹ für -H steht.

6. Verfahren nach einem der Ansprüche 1 bis 5,
wobei R¹, R³, R⁴, R⁵, R⁶, R⁷, R⁸ jeweils für-H stehen.

7. Verfahren nach einem der Ansprüche 1 bis 6,
wobei das Amin in Verfahrensschritt a) eine Struktur gemäß der allgemeinen Formel II aufweist: 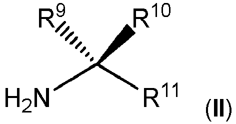 und
R⁹, R¹⁰, R¹¹ jeweils unabhängig voneinander ausgewählt sind aus: -H, -(C₁-C₁₂)-Alkyl,-O-(C₁-C₁₂)-Alkyl, -(C₃-C₁₂)-Cycloalkyl, Phenyl;
und die Alkyl-, Cycloalkyl-, Phenyl-Reste jeweils unabhängig voneinander mit einem oder mehreren Substituenten ausgewählt aus: -(C₁-C₁₂)-Alkyl, -(C₃-C₁₂)-Cycloalkyl, -O-(C₁-C₁₂)-Alkyl, -O-(C₃-C₁₂)-Cycloalkyl, substituiert sein können,
und sich jeder der drei Reste R⁹, R¹⁰, R¹¹ jeweils von den beiden anderen Resten unterscheidet.

8. Verfahren nach einem der Ansprüche 1 bis 7,
wobei R¹¹ für einen Phenylrest steht.

9. Verfahren nach einem der Ansprüche 1 bis 8,
wobei R¹⁰ für -(C₁-C₁₂)-Alkyl steht.

10. Verfahren nach einem der Ansprüche 1 bis 9,
wobei R⁹ für -H steht.

11. Verfahren nach einem der Ansprüche 1 bis 10,
wobei im Verfahrensschritt b) als Anode und Kathode jeweils eine Glaskohlenstoffelektrode verwendet wird.

12. Verfahren nach einem der Ansprüche 1 bis 11,
wobei als Lösungsmittel im Verfahrensschritt b) 1,1,1,3,3,3-Hexafluorisopropanol verwendet wird.

13. Verfahren nach einem der Ansprüche 1 bis 12,
wobei als Leitsalz im Verfahrensschritt b) Tributylmethylammoniummethylsulfat verwendet wird.

14. Verfahren nach einem der Ansprüche 1 bis 13,
wobei das Phenol in Verfahrensschritt b) eine Struktur gemäß der allgemeinen Formel **III** aufweist: 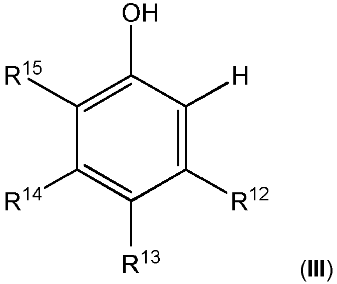 und
R¹², R¹³, R¹⁴, R¹⁵ jeweils unabhängig voneinander ausgewählt sind aus: -H, -(C₁-C₁₂)-Alkyl, -O-(C₁-C₁₂)-Alkyl, -(C₃-C₁₂)-Cycloalkyl, Phenyl;
und die Alkyl-, Cycloalkyl-, Phenyl-Reste jeweils unabhängig voneinander mit einem oder mehreren Substituenten ausgewählt aus: -(C₁-C₁₂)-Alkyl, -(C₃-C₁₂)-Cycloalkyl, -O-(C₁-C₁₂)-Alkyl, -O-(C₃-C₁₂)-Cycloalkyl, substituiert sein können.

15. Verbindung, welche eine der folgenden Formel aufweist:

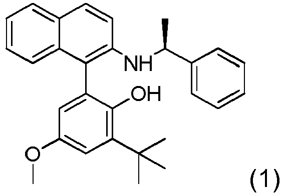

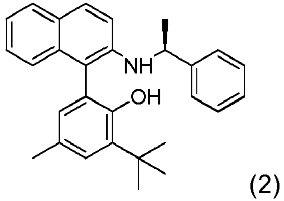

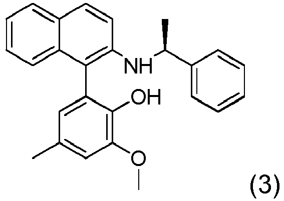

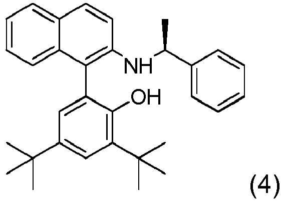

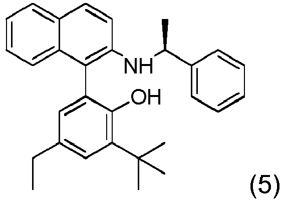

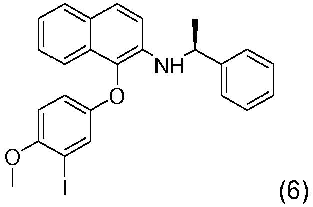

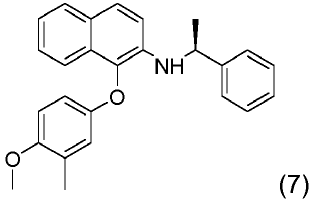

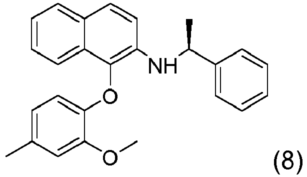

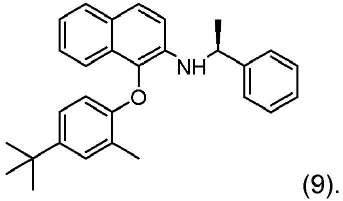

## Claims

1. Method comprising the method steps of:
a) reacting a naphthol, which has a hydrogen in the position ortho to the OH group, with a chiral primary amine in the presence of water and with addition of sodium hydrogen sulphite such that a secondary naphthylamine derivative is formed,
b) electrochemical coupling of the secondary naphthylamine derivative from a), using a solvent and a conductive salt, with a phenol which has a hydrogen in the position ortho to the OH group, such that
- either the phenyl ring of the phenol in the position ortho to the OH group is directly coupled to the naphthyl ring system in the position ortho to the amino group,
or
- the oxygen of the phenol is coupled to the naphthyl ring system in the position ortho to the amino group.

2. Method according to Claim 1,
wherein the reaction in method step a) is conducted at a temperature in the range of 80°C to 120°C.

3. Method according to Claim 1 or 2,
wherein the sodium hydrogen sulphite in method step a) is added in an amount of 2 to 8 equivalents, based on the naphthol.

4. Method according to any of Claims 1 to 3,
wherein the naphthol in method step a) has a structure according to the general formula **I**: 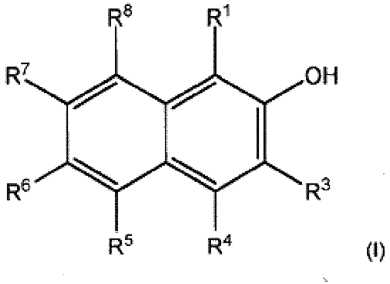 and
R¹, R³, R⁴, R⁵, R⁶, R⁷, R⁸ are each independently selected from : -H, -(C₁-C₁₂)-alkyl, -O-(C₁-C₁₂)-alkyl, -(C₃-C₁₂)-cycloalkyl,
at least one of the radicals R¹, R³ is -H,
and the alkyl and cycloalkyl radicals may each independently be substituted by one or more substituents selected from: -(C₁-C₁₂)-alkyl, -(C₃-C₁₂)-cycloalkyl, -O-(C₁-C₁₂)-alkyl, -O-(C₃-C₁₂)-cycloalkyl.

5. Method according to any of Claims 1 to 4,
wherein R¹ is -H.

6. Method according to any of Claims 1 to 5,
wherein R¹, R³, R⁴, R⁵, R⁶, R⁷, R⁸ are each -H.

7. Method according to any of Claims 1 to 6,
wherein the amine in method step a) has a structure according to the general formula **II**: 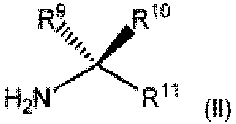 and
R⁹, R¹⁰, R¹¹ are each independently selected from: -H, -(C₁-C₁₂)-alkyl, -O-(C₁-C₁₂)-alkyl, -(C₃-C₁₂) cycloalkyl, phenyl;
and the alkyl, cycloalkyl and phenyl radicals may each independently be substituted by one or more substituents selected from: -(C₁-C₁₂)-alkyl, -(C₃-C₁₂)-cycloalkyl, -O-(C₁-C₁₂)-alkyl, -O-(C₃-C₁₂)-cycloalkyl,
and each of the three radicals R⁹, R¹⁰, R¹¹ in each case differ from the two other radicals.

8. Method according to any of Claims 1 to 7,
wherein R¹¹ is a phenyl radical.

9. Method according to any of Claims 1 to 8,
wherein R¹⁰ is -(C₁-C₁₂)-alkyl.

10. Method according to any of Claims 1 to 9,
wherein R⁹ is -H.

11. Method according to any of Claims 1 to 10,
wherein a glassy carbon electrode is used in each case as anode and cathode in method step b).

12. Method according to any of Claims 1 to 11,
wherein the solvent used in method step b) is 1,1,1,3,3,3-hexafluoroisopropanol.

13. Method according to any of Claims 1 to 12,
wherein the conductive salt used in method step b) is tributylmethylammonium methyl sulfate.

14. Method according to any of Claims 1 to 13,
wherein the phenol in method step b) has a structure according to the general formula **III**: 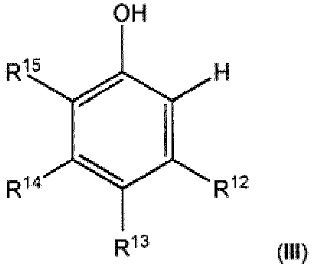 and
R¹², R¹³, R¹⁴, R¹⁵ are each independently selected from: -H, -(C₁-C₁₂)-alkyl, -O-(C₁-C₁₂)-alkyl, -(C₃-C₁₂)-cycloalkyl, phenyl;
and the alkyl, cycloalkyl and phenyl radicals may each independently be substituted by one or more substituents selected from: -(C₁-C₁₂)-alkyl, -(C₃-C₁₂)-cycloalkyl, -O-(C₁-C₁₂)-alkyl, -O-(C₃-C₁₂)-cycloalkyl.

15. Compound having one of the following formulae:

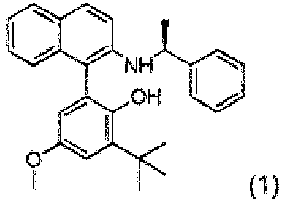

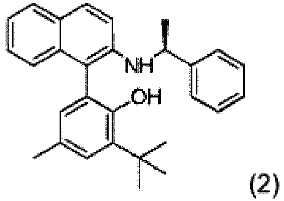

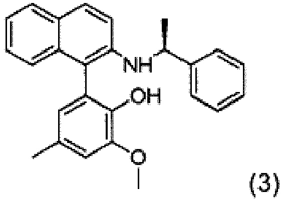

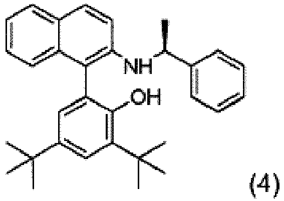

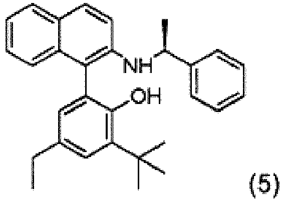

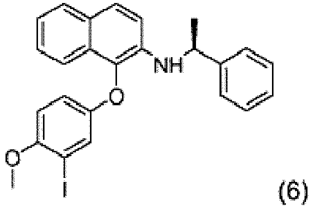

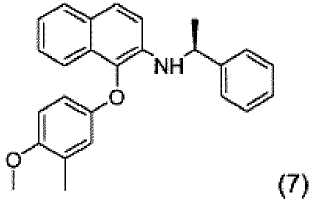

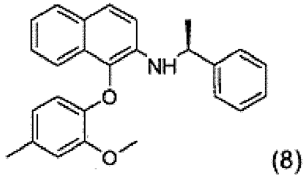

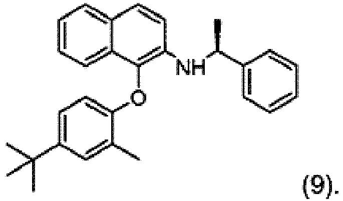

## Revendications

1. Procédé comprenant les étapes de procédé suivantes :
a) la mise en réaction d'un naphtol, qui comprend en position ortho du groupe OH un hydrogène, avec une amine primaire chirale en présence d'eau et avec ajout d'hydrogénosulfite de sodium, de manière à former un dérivé de naphtylamine secondaire,
b) le couplage électrochimique du dérivé de naphtylamine secondaire de a) en utilisant un solvant et un sel conducteur avec un phénol, qui comprend en position ortho du groupe OH un hydrogène, de sorte que
- soit un couplage direct du cycle phényle du phénol en position ortho du groupe OH avec le système cyclique naphtyle en position ortho du groupe amino ait lieu,
- soit l'oxygène du phénol soit couplé au système cyclique naphtyle en position ortho du groupe amino.

2. Procédé selon la revendication 1, dans lequel la réaction à l'étape de procédé a) a lieu à une température dans la plage allant de 80 °C à 120 °C.

3. Procédé selon la revendication 1 ou 2, dans lequel l'hydrogénosulfite de sodium à l'étape de procédé a) est ajouté en une quantité de 2 à 8 équivalents par rapport au naphtol.

4. Procédé selon l'une quelconque des revendications 1 à 3, dans lequel le naphtol à l'étape de procédé a) présente une structure selon la formule générale I : 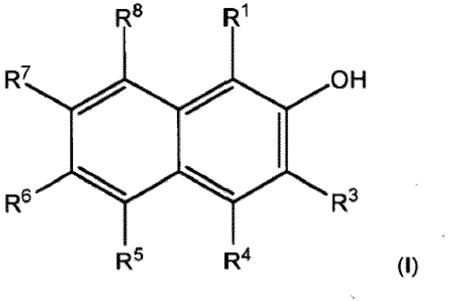 et
R¹, R³, R⁴, R⁵, R⁶, R⁷, R⁸ sont chacun choisis indépendamment les uns des autres parmi : -H, -alkyle en (C₁-C₁₂), -O-alkyle en (C₁-C₁₂), -cycloalkyle en (C₃-C₁₂), au moins un des radicaux R¹, R³ représente -H,
et les radicaux alkyle et cycloalkyle peuvent chacun être substitués indépendamment les uns des autres avec un ou plusieurs substituants choisis parmi : -alkyle en (C₁-C₁₂), -cycloalkyle en (C₃-C₁₂), -O-alkyle en (C₁-C₁₂), -O-cycloalkyle en (C₃-C₁₂).

5. Procédé selon l'une quelconque des revendications 1 à 4, dans lequel R¹ représente -H.

6. Procédé selon l'une quelconque des revendications 1 à 5, dans lequel R¹, R³, R⁴, R⁵, R⁶, R⁷, R⁸ représentent chacun -H.

7. Procédé selon l'une quelconque des revendications 1 à 6 dans lequel l'amine à l'étape de procédé a) présente une structure selon la formule générale II : 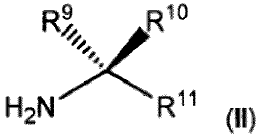 et
R⁹, R¹⁰, R¹¹ sont chacun choisis indépendamment les uns des autres parmi : -H, -alkyle en (C₁-C₁₂), -O-alkyle en (C₁-C₁₂), -cycloalkyle en (C₃-C₁₂), phényle ;
et les radicaux alkyle, cycloalkyle, phényle peuvent chacun être substitués indépendamment les uns des autres avec un ou plusieurs substituants choisis parmi : -alkyle en (C₁-C₁₂), -cycloalkyle en (C₃-C₁₂), -O-alkyle en (C₁-C₁₂), -O-cycloalkyle en (C₃-C₁₂),
et chacun des trois radicaux R⁹, R¹⁰, R¹¹ diffère des deux autres radicaux.

8. Procédé selon l'une quelconque des revendications 1 à 7, dans lequel R¹¹ représente un radical phényle.

9. Procédé selon l'une quelconque des revendications 1 à 8, dans lequel R¹⁰ représente -alkyle en (C₁-C₁₂).

10. Procédé selon l'une quelconque des revendications 1 à 9, dans lequel R⁹ représente -H.

11. Procédé selon l'une quelconque des revendications 1 à 10, dans lequel, à l'étape de procédé b), une électrode de carbone vitreux est utilisée à chaque fois en tant qu'anode et cathode.

12. Procédé selon l'une quelconque des revendications 1 à 11, dans lequel du 1,1,1,3,3,3-hexafluoroisopropanol est utilisé en tant que solvant à l'étape de procédé b).

13. Procédé selon l'une quelconque des revendications 1 à 12, dans lequel du méthylsulfate de tributylméthylammonium est utilisé en tant que sel conducteur à l'étape de procédé b).

14. Procédé selon l'une quelconque des revendications 1 à 13, dans lequel le phénol à l'étape de procédé b) présente une structure selon la formule générale III : 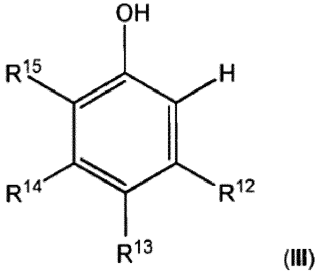 et
R¹², R¹³, R¹⁴, R¹⁵ sont chacun choisis indépendamment les uns des autres parmi : -H, -alkyle en (C₁-C₁₂), -O-alkyle en (C₁-C₁₂), -cycloalkyle en (C₃-C₁₂), phényle ;
et les radicaux alkyle, cycloalkyle, phényle peuvent chacun être substitués indépendamment les uns des autres avec un ou plusieurs substituants choisis parmi : -alkyle en (C₁-C₁₂), -cycloalkyle en (C₃-C₁₂), -O-alkyle en (C₁-C₁₂), -O-cycloalkyle en (C₃-C₁₂).

15. Composé, qui présente une des formules suivantes :
